# EUROPÄISCHE PATENTSCHRIFT

(11) **EP 1 254 166 B1**
(45) Veröffentlichungstag und Bekanntmachung des Hinweises auf die Patenterteilung: **03.09.2008**
(21) Anmeldenummer: 01909609.8
(22) Anmeldetag: 11.01.2001
(51) Int. Cl.: C07K 14/00

(54) **REKOMBINANTE HERSTELLUNG VON HUMANEN HISTON 1-SUBTYPEN SOWIE DEREN VERWENDUNG FÜR THERAPEUTISCHE ZWECKE**
RECOMBINANT PRODUCTION OF HUMAN HISTONE-1 SUBTYPES AND USE THEREOF FOR THERAPEUTIC PURPOSES
PRODUCTION RECOMBINANTE DE SOUS-TYPES D'HISTONE-1 HUMAINE ET LEUR UTIILSATION A DES FINS THERAPEUTIQUES

(30) Priorität: 13.01.2000 DE 10001113
(43) Veröffentlichungstag der Anmeldung: 06.11.2002
(73) Patentinhaber: Richter-HELM BioLogics GmbH & Co. KG, 24796 Bovenau (DE)
(72) Erfinder: POHLMEYER, Kai, 25579 Fitzbek (DE); BEHNKE, Bert, 22926 Ahrensburg (DE); WICK, Ralf, 22529 Hamburg (DE); MAYER, Gerd, 22529 Hamburg (DE)
(74) Vertreter: Neuefeind, Regina
(86) Internationale Anmeldenummer: PCT/EP2001/000290
(87) Internationale Veröffentlichungsnummer: WO 2001/051511

(56) Entgegenhaltungen:
- BURNETT VICKI L ET AL: "High-level expression of human histone H4 in E. coli." BIOTECHNIQUES, Bd. 26, Nr. 1, Januar 1999 (1999-01), Seite 30, 32, 34 XP000999268 ISSN: 0736-6205
- WELLMAN SUSAN E ET AL: "Purification of mouse HI histones expressed in Escherichia coli." BIOTECHNOLOGY AND APPLIED BIOCHEMISTRY, Bd. 26, Nr. 2, 1997, Seiten 117-123, XP000886236 ISSN: 0885-4513 in der Anmeldung erwähnt
- DOENECKE D ET AL: "DIFFERENTIAL DISTRIBUTION OF LYSINE AND ARGININE RESIDUES IN THE CLOSELY RELATED HISTONES H-10 AND H-5 ANALYSIS OF A HUMAN H-10 GENE" JOURNAL OF MOLECULAR BIOLOGY, Bd. 187, Nr. 3, 5. Februar 1986 (1986-02-05), Seiten 461-464, XP001005460 ISSN: 0022-2836
- EICK S ET AL: "HUMAN H1 HISTONES CONSERVED AND VARIED SEQUENCE ELEMENTS IN TWO H1 SUBTYPE GENES" EUROPEAN JOURNAL OF CELL BIOLOGY, Bd. 49, Nr. 1, Juni 1989 (1989-06), Seiten 110-115, XP001005459 ISSN: 0171-9335
- POHLMEYER KAI ET AL: "The recombinant human histones H1degree and H1.2 cause different toxicity profiles on the human leukemia cell line K562." ANTICANCER RESEARCH, Bd. 20, Nr. 4, Juli 2000 (2000-07), Seiten 2499-2504, XP001000135 ISSN: 0250-7005

### SEQUENZPROTOKOLL

(1) ALLGEMEINE ANGABEN:
   (i) ANMELDER:
      (A) NAME: Strathmann AG & Co.
      (B) STRASSE: Sellhopsweg 1
      (C) ORT: Hamburg
      (E) LAND: Deutschland
      (F) POSTLEITZAHL: 22459
   (ii) BEZEICHNUNG DER ERFINDUNG: Rekombinante Herstellung von humanen Histon 1-Subtypen sowie deren Verwendung für therapeutische Zwecke
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   ii) ART DES MOLEKÜLS: Sonstige Nucleinsäure
      (A) BESCHREIBUNG: /desc = "Oligonukleotidprimer"
   (xi) SEQUENZBESCHREIBUNG: SEQ ID NO: 1:
      GGGGGGGGAT CCATATGACC GAGAATTCCA CGTCCGCCC 39
(2) ANGABEN ZU SEQ ID NO: 2:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   ii) ART DES MOLEKÜLS: Sonstige Nucleinsäure
      (A) BESCHREIBUNG: /desc = "Oligonukleotidprimer"
   (xi) SEQUENZBESCHREIBUNG: SEQ ID NO: 2:
      GGGGGGGTCG ACTCACTTCT TCTTGCCGGC CCTCTTGGC 39
(2) ANGABEN ZU SEQ ID NO: 3:
   (i) SEQUENZKENNZEICHEN:
      (A) LÄNGE: 37 Basenpaare
      (B) ART: Nucleotid
      (C) STRANGFORM: Einzelstrang
      (D) TOPOLOGIE: linear
   ii) ART DES MOLEKÜLS: Sonstige Nucleinsäure
      (A) BESCHREIBUNG: /desc = "Oligonukleotidprimer"
   (xi) SEQUENZBESCHREIBUNG: SEQ ID NO: 3:
      GGGGGGGGAT CCATATGTCC GAGACTGCTC CTGCCGC 37
(2) ANGABEN ZU SEQ ID NO: 4:
   (i) SEQUENZKENNZEICHEN:
      (A) LÄNGE: 37 Basenpaare
      (B) ART: Nucleotid
      (C) STRANGFORM: Einzelstrang
      (D) TOPOLOGIE: linear
   ii) ART DES MOLEKÜLS: Sonstige Nucleinsäure
      (A) BESCHREIBUNG: /desc = "Oligonukleotidprimer"
   (xi) SEQUENZBESCHREIBUNG: SEQ ID NO: 4:
      GGGGGGGTCG ACCTATTTCT TCTTGGGCGC CGCCTTC 37

## Beschreibung

Die Erfindung betrifft rekombinant hergestellte humane Histon 1-Subtypen sowie deren Verwendung für therapeutische Zwecke.

Das Protein Histon 1 und dessen verschiedene Subtypen, wie beispielsweise das Histon 1° und das Histon 1.2, sind neben weiteren Histonen zusammen mit DNA die Hauptkomponenten von Chromatin. Sie spielen eine wesentliche Rolle bei der Organisation der Chromatinstruktur sowie bei der Genrepression.

Des weiteren ist bekannt, dass die Histone Funktionen außerhalb des Zellkerns bzw. der Zelle besitzen. So können Lymphozyten der Milz und des Thymus im Rahmen der Apoptose Histone in den Kreislauf sekretieren (D.A. Bell et al (1990), J. Clin. Invest. 85, 1487-1496). Ferner sind in Seeigeleiern H1-Histone integrale Komponenten des Cytoplasmas (Multigner, L., Gagnon, J., Van Dorsselaer, A., Job, D. (1992) Nature 360, 33-39). Des weiteren wurde berichtet, dass extrazellulär auftretendes Histon H1 als Rezeptor von Thyroglobin, einem hormonell wirksamen Glycoprotein aus der Schilddrüse, auf der Plasmamembran von Makrophagen der Maus dienen kann (Brix, K., Summa, W., Lottspeich, F., Herzog, V. (1998) J. Clin. Invest. 102, 283-293). Zelloberflächenhistone sind die Ursache für die Bildung von Autoantikörpern bei Autoimmunkrankheiten wie dem SLE-Syndrom (systematischer Lupus erythematodes) (Holers, V.M., and Kotzin, B.L. (1985) J. Clin. Invest.76, 991-998).

Ferner ist bekannt, dass die Histone nicht nur in vitro (Hirsch, J.D. (1958) J. Exp. med. 108, 925-944), sondern auch im humanen Gastrointestinaltrakt (Rose, F.R.A.J., Bailey, K., Keyte, J.W., Chan, W.C., Greenwood, D., Mahida, Y.R. (1998) Infectin and Immunity 66, 3255-3263) oder auf der Haut von Fischspezies (Robinette, d., Wada, S., Arroll, T., Levy, m.G., Miller, W.L. Noga, E.J. (1998) Cell. Mil. Life Sci. 54, 467-475) antimikrobielle Wirkungen aufweisen. Diese antimikrobielle Aktivität der Histone beruht auf der Gegenwart N-terminaler Peptide mit antimikrobieller Wirkung wie Buforin 1, dargestellt durch die aminoterminalen 39 Aminosäuren des Histons H2A (Kim, H.S., Park, C.B., Kim, M.S., Kim, S.C. (1996) Biochem. Biophys. Res. Commun. 10, 940-948), oder Parasin, das ebenfalls ein N-terminales Spaltprodukt des Histons H2A ist.

Eine wesentliche Bedeutung für therapeutische Anwendungen haben die Histone durch ihre Wirkung als Cytostatika für eukaryotische Zellen erlangt. So ist bekannt, dass durch gereinigtes Rinderhiston 1 die Lebensfähigkeit von verschiedenen Leukämie-Zelllinien sowohl in vitro als auch das Tumorwachstum in vivo unterdrückt werden kann (Class, R., Lindman, S., Fassbender, C., Leinenbach, H.-P., Rawer, S., Emrich, J.G., Grady, L.W., Zeppezauer, M. (1996) Am. J.Clin. Oncol. 19(5), 522-531). Gemäß dieser Veröffentlichung wirkt das gereinigte Rinderhiston 1 jedoch nicht toxisch auf nicht transformierte PBMC (peripheral blood mononuclear cells). Dies legt nahe, dass die PBMC erst nach einer Transformation in Tumorzellen durch Histone angegriffen werden können.

In Biotechnol. Appl. Biochem. (1997, 26, 117-123) wird die Reinigung von in *E. coli* exprimierten H1-Histonen der Maus beschrieben. Eine Expression von humanen H1-Histonen wird dort nicht angesprochen.

Die heterologe Expression von humanen H1-Histonen in Hefe beschreiben W. Albig et al. in FEBS Letters (1998), 435, 245-250; C. Saunders und R. S. Cohen berichteten über die Expression von humanem Histon H4 in E. coli (Biotechnics 1999, 26, 30-34).

In dem europäischen Patent EP-B1-0 392 315 ist die Verwendung von reinem Histon H 1 für therapeutische Zwecke, insbesondere als Cystostatikum, offenbart. Gemäß dieser Druckschrift wurden die verwendeten H1-Histone aus Thymuszellen des Rindes isoliert.

Es ist davon auszugehen, dass humane Histone für die therapeutische Anwendung am Menschen besser geeignet sind als Histone beispielsweise des Rindes oder der Maus.

Ferner ist es vorteilhaft, derartige Histone durch rekombinante Herstellungsverfahren bereitzustellen, da derartige Verfahren angesichts der für die therapeutische Verwendung benötigten Mengen wesentlich effizienter und kostengünstiger sind als beispielsweise eine aufwendige Isolierung von Histonen aus dem Thymus des Menschen oder des Rindes.

Abgesehen davon, dass eine rekombinante Herstellung die Validierung und Überwachung des Herstellungsprozesses wesentlich erleichtert, bietet die Herstellung in gentechnisch veränderten Organismen bedeutende Vorteile im Hinblick auf Virussicherheit und Inprozeß-Kontrollen.

Demzufolge ist es eine wesentliche Aufgabe der Erfindung, humane Histontypen oder -subtypen bereitzustellen, die durch ein effizientes biotechnologisches Verfahren rekombinant hergestellt werden können.

Des weiteren ist es eine wichtige Aufgabe der Erfindung, Möglichkeiten der therapeutischen Verwendung der erfindungsgemäßen Histontypen aufzuzeigen.

Insbesondere ist es eine Aufgabe der vorliegenden Erfindung, humane Histonsubtypen bereitzustellen, die bei geringer Dosis einen hohe Cytotoxizität auf Tumorzelllinien aufweisen.

Weitere Aufgaben ergeben sich aus der folgenden Beschreibung.

Diese Aufgaben werden durch die Gegenstände der unabhängigen Ansprüche, insbesondere basierend auf der Bereitstellung der erfindungsgemäßen Histonsubtypen H1° und H1.2, gelöst.

Vorteilhafte Ausgestaltungen sind in den Unteransprüchen beschrieben.

Die vorliegende Erfindung betrifft somit rekombinant in prokaryoten Zellen, insbesondere in *E*. *coli* hergestellte Proteine mit der biologischen Aktivität des humanen Histon 1 oder eines aktiven Fragments davon. Ein biologisch aktives Fragment bedeutet im Zusammenhang mit dieser Erfindung, dass die vermittelte biologische Aktivität zu einer therapeutischen Anwendung ausreicht. Insbesondere betrifft die Erfindung rekombinant hergestellte humane Histon 1-Proteine des Subtyps H1° oder H1.2.

Erfindungsgemäß werden die humanen H1-Proteine durch ein Verfahren hergestellt, das folgende Schritte umfasst:
a) Expression einer für humanes Protein Histon 1 kodierenden Nukleinsäuresequenz in prokaryoten Zellen, insbesondere *E*. *coli* und
b) Extraktion und Reinigung des rekombinanten humanen Proteins Histon 1 aus den prokaryoten Zellen, insbesondere *E. coli.*

Es ist zu erwähnen, dass es angesichts der starken antimikrobiellen Wirkung der Histone als äußerst überraschend anzusehen ist, dass überhaupt eine Expression und besonders eine effiziente Expression von Histonen in Bakterien möglich ist. Dieses Ergebnis konnte bei Kenntnis des Standes der Technik, siehe z.B. Hirsch, J.D., 1958, vide supra, nicht erwartet werden.

Eine weitere Aufgabe der Erfindung besteht darin, Verwendungen der erfindungsgemäßen humanen Histone H1, insbesondere von H1° und H1.2, für therapeutische Zwecke aufzuzeigen.

Gegenstand der Erfindung ist dabei insbesondere die Verwendung der erfindungsgemäßen Histone zur Krebstherapie. Dazu zählt beispielsweise die Verwendung der erfindungsgemäßen Histone zur Therapie von Karzinomen, Melanomen, Sarkomen, Mesotheliomen sowie von malignen Erkrankungen, insbesondere von solchen des lymphatischen Systems, die durch maligne B- und T-Zellen, wie B-lymphoblastisches Lymphom, myelogenisches Lymphom oder Burkitt-Lymphom, verursacht werden.

Bei der Untersuchung der cytostatischen Effekte der erfindungsgemäßen humanen Histone H1° und H1.2 wurde aus Rindern isoliertes und gereinigtes Histon 1 als geeignete Vergleichsprobe herangezogen, da die Histone sehr konservierte Moleküle sind. Beispielsweise zeigen humanes Histon H4 und Rinderhiston H4 eine Homologie von über 90%. Maushiston H1.2 und humanes Histon H1.2 zeigen eine Homologie von über 97% und über 88% Identität. Leider sind keine entsprechenden Daten über die H1°- oder H1.2 Sequenzen von Rindern erhältlich.

Bei der erfindungsgemäßen Verwendung der erfindungsgemäßen humanen Histone H1° und H1.2 zur Behandlung von Tumorzelllinien wurden zwei überraschende Beobachtungen gemacht:

Zum einen wurde gefunden, dass die erfindungsgemäßen humanen rekombinanten Histone H1° und H1.2 sowie das konventionell gewonnene Rinderhiston 1 cytotoxische Effekte auf gesunde PBMCs aufweisen, die sich von den bislang beschriebenen Effekten unterscheiden. So wurde beispielsweise im Stand der Technik berichtet, dass gereinigtes Rinderhiston 1 zwar cytotoxisch auf eine Reihe von Leukämie-Zelllinien wirkt, jedoch keine Effekte auf nicht in Tumorzellen transformierte PBMCs hat (Class, R. *et al.,* vide supra). Im Gegensatz dazu wurde nun gefunden, dass die erfindungsgemäßen humanen rekombinanten Histone H1° und H1.2 sowie das Rinderhiston 1 eine zeit- und dosisabhängige Cytotoxizität sowohl auf Leukämie-Zelllinien als auch auf PBMCs aufweisen.

Während die Toxizität auf die Leukämiezellen unmittelbar nach Zugabe der erfindungsgemäßen Histone auftritt, ist eine signifikante Toxizität auf PBMCs erst nach einer Dauer von mindestens einer Stunde beobachtbar. Nach 24 Stunden Inkubation zeigen die rekombinanten humanen Histone H1° und H1.2 sowie die Vergleichsprobe Rinderhiston 1 bei einer Dosis von 250 µg/ml jedoch eine Toxizität von über 50%.

Das erfindungsgemäße rekombinante humane Histon H1.2 weist cytotoxische Effekte auf Tumorzellen, insbesondere auf Leukämiezelllinien auf, die hinsichtlich der Dosis- und Zeitabhängigkeit ähnlich zu denen des Rinder-H1-Histons sind.

Während die cytotoxischen Effekte von humanem Histon H1° auf PBMCS hinsichtlich der Dosis- und Zeitabhängigkeit ähnlich zu denen des humanen Histon 1.2 als auch der Vergleichsprobe Rinderhiston H1 sind, wurde nun als weiterer wesentlicher Aspekt der Erfindung überraschenderweise gefunden, dass das erfindungsgemäße humane rekombinante H1° weniger toxisch auf Tumorzellen, insbesondere auf Leukämiezellen ist als das erfindungsgemäße humane rekombinante H1.2 und das Rinderhiston 1. Ein derartiger Unterschied in den cytotoxischen Effekten von Histonsubtypen eines Histons wurde bislang noch nicht beschrieben und war angesichts der Sequenzhomologie der humanen Histonsubtypen H1° und H1.2 nicht zu erwarten.

Angesichts der vorstehend erstmals beschriebenen zeitabhängigen Toxizität der Histone auf PBMCs von gesunden Spendern ist es für die Verwendung von humanem Histon 1 zur Behandlung von Tumoren von besonderer Bedeutung, Histon 1-Subtypen zu verwenden, die bereits bei geringer Dosis toxisch auf maligne Zellen wirken. Dass sich verschiedene Histonsubtypen eines Histons hinsichtlich ihrer Cytotoxizität unterscheiden können, war bislang noch nicht bekannt. Somit wird durch die vorliegende Erfindung mit dem erfindungsgemäßen humanen Histon H1.2 erstmals ein Histon 1-Subtyp bereitgestellt, der für die Verwendung zur Krebstherapie aufgrund seiner Cytotoxizitätseigenschaften sowie aufgrund seiner humanen Sequenz besonders vorteilhaft geeignet ist.

Ohne an eine Hypothese gebunden sein zu wollen, wird für die unterschiedlichen Toxizitäten der erfindungsgemäßen Histone H1° und H1.2 auf Tumorzelllinien gegenwärtig folgende Erklärung angenommen: Es wurde gezeigt, dass Histone und andere basische Proteine leitfähige Kanäle in verschiedenen Membranen bilden können (Kleine, T.J., Lewis, P.N., Lewis, S.A. (1997) Am. J. Physiol. 273, C1925-C 1936; Gamberucci, A., Fulceri, R., Marcolongo, P., Pralong, W.F., Benedetti, A. (1996) Biochem. J. 331, 623-630; Kleine, T.J., Gladfelter, A., Lewis, P.N., Lewis, S.A. (1995) Am. J. Physiol. 268, C1114-C1125). Durch die Kanalbildung wird die Durchlässigkeit der Zellmembran für kleine monovalente Kationen und Anionen erhöht, und es wird angenommen, daß diese erhöhte Permeabilität zu einem Anschwellen der Zelle und schließlich zur Zelllyse führt.

Möglich wäre, dass die erhöhte Kanalaktivität eine Folge einer erhöhten Stabilität der Bindung der Histone an die negativ geladene Phospholipidmembran ist. Da jedoch die Histone H1° und H1.2 eine sehr ähnliche Gesamtladung aufweisen (H1°: 61 Aminosäuren positiv geladen, 7 Aminosäuren negativ geladen; H1.2: 62 Aminosäuren positiv geladen, 7 Aminosäuren negativ geladen), scheint es unwahrscheinlich, dass die stärkere Cytotoxizität des Histon H1.2 auf einer höheren Affinität für die negativ geladene Zellmembran beruht.

Bevor die Kanalbildung erfolgt, ist die Erkennung und die Bindung an die Membran erforderlich. Es wurde vorgeschlagen, dass dies durch einen Histon H1-Rezeptor auf der Membran der malignen Zellen erfolgt. Daher wird angenommen, dass die unterschiedliche Cytotoxizität von H1.2 und H1° darauf beruht, dass auf der Zelloberfläche ein Histonrezeptor mit einer stärkeren Affinität für Histon H1.2 vorliegt. Unter der Annahme ähnlicher Bindungscharakteristika könnten die unterschiedlichen Toxizitäten nur durch unterschiedliche Kanaleigenschaften von H1° und H1.2 erklärt werden.

Gegenstand der Erfindung ist ebenfalls die Verwendung der erfindungsgemäßen humanen Histone H1 als antimikrobielle Wirkstoffe, insbesondere als Chemotherapeutika und Antibiotika. Die erfindungsgemäßen Histone sind insbesondere wirksam gegen einen breiten Bereich von Mikroorganismen, einschließlich Bakterien, Viren, Pilzen sowie Parasiten.

Weiter betrifft die Erfindung die Verwendung der erfindungsgemäßen Histone zur Therapie von endokrinen Störungen. Die erfindungsgemäßen Histone können ebenfalls vorteilhafterweise zur Therapie von Immunkrankheiten, insbesondere zur Therapie von Autoimmunkrankheiten wie beispielsweise dem SLE-Syndrom verwendet werden.

Die vorliegende Erfindung umfasst somit jede mögliche Form der Verwendung der erfindungsgemäßen rekombinant in prokaryoten Zellen, insbesondere E. *coli,* hergestellten humanen Histone H1, bevorzugt der Histone H1° und H 1.2, insbesondere bevorzugt des Histon H1.2, deren biologische Aktivität eine therapeutische Wirkung herbeiführt.

Die folgenden Beispiele dienen der Erläuterung der Erfindung, ohne diese in irgendeiner Weise einzuschränken.

### Beispiele:

### Beispiel 1: Isolierung der Histone H1° und H1.2 aus E. coli

a) cDNA-Klonierung und Expression: cDNAs für die Histone H1° und H1.2 wurden aus Gesamt-RNA von HepG₂-Zellen (Aden et al. (1979) Nature 283:615-616; Know les et al. (1980) Science 209:497-499) durch RT-PCR erhalten. Dazu wurden die HepG₂-Zellen in RPMI 1640 (Biochrom, Berlin) mit 10% FCS (Biowhittaker, Verviers, Belgien) gezüchtet. Die Gesamt-RNA wurde mit Trizol (Gibco BRL, Rockville, MD) entsprechend der Empfehlung der Hersteller isoliert. Die reverse Transkription wurde mit 2 µg der Gesamt-RNA und 500 ng Oligo[dT30]-Primer unter den durch den Hersteller (Gibco BRL) empfohlenen Bedingungen durchgeführt. 1 µl der cDNA wurde für die RT-PCR mit den folgenden Primern verwendet: 
H1° (forward): 5'
GGGGGGGATCCATATGACCGAGAATTCCACGTCCGCCC 3'
 
H1° (reverse): 5'
GGGGGGGTCGACTCACTTCTTCTTGCCGGCCCTCTTGGC 3'
 
H1.2 (forward): 5' GGGGGGGGATCCATATGTCCGAGACTGCTCCTGCCGC
3'
 
H1.2 (reverse): 5' GGGGGGGTCGACCTATTTCTTCTTGGGCGCCGCCTTC
3'
   Die PCR umfasste einen 5-minütigen Denaturierungsschritt bei 94°C, gefolgt von 35 Zyklen von jeweils 1 Minute bei 94°C, 1 Minute bei 52°C und 1 Minute bei 72°C und einem abschließenden Verlängerungsschritt von 10 Minuten bei 72°C.
   Der für H1° kodierende Klon stimmte zu 100% mit der Sequenz der Accession No. X03473 (D. Dönecke, R. Tonjes (1986), J. Mol. Biol., 187 (3), 461-464) überein, und der für H 1.2 kodierende Klon stimmte zu 100% mit der Sequenz der Accession No. X57129 (S. Eick et al. (1989), Eur. J. Cell Biol. 49 (1), 110-115) überein. Die PCR-Produkte wurden in den Vektor pET24 (a) (Novagen, Madison, USA) kloniert und durch Sequenzierung nach Sanger überprüft. Beide Klone wurden in *E. coli* BL21 (DE3) exprimiert.
b) Extraktion von H1° und H1.2 aus *E. coli:* Nach 2 Stunden Induktion mit IPTG wurden die Bakterien geerntet, durch Druck lysiert und einer Zentrifugation bei 11.000 x g unterzogen. Die Überstände wurden verworfen und die Histonenthaltenden Pellets wurden mit 0,75 M Perchlorsäure extrahiert. Die Extraktionen wurden für eine Dauer von 15 Minuten auf Eis unter kontinuierlichem Rühren durchgeführt. Die Mischung wurde durch Zentrifugation (11.000 x g) und eine nachfolgende Filtration durch eine 0,45 µm-Zelluloseacetatmembran (Sartorius, Göttingen, Deutschland) gereinigt. Die klaren Lysate wurden direkt der HPLC unterzogen.
c) Reversed-phase Chromatographie: Zu den sauren Histon-Extrakten wurde Methanol bis zu einer Menge von 10% des Endvolumens zugegeben, und die Extrakte wurden auf eine Reversed-phase C18 (5 µm)-Säule aufgetragen. Nach dem Waschen mit 5 Volumenteilen des Puffers A (0,1% Trifluoressigsäure, TFA) wurden die H1-Histone durch einen steigenden Acetonitril-Wasser-Gradienten getrennt. Das H1°-Histon wurde üblicherweise im Bereich von 20-35% Acetonitril eluiert, während H1.2 bei 25-45% Acetonitril eluiert wurde. Die Reinheit der Histonfraktionen wurde durch SDS-PAGE nach Lämmli und analytische HPLC, die auf einer Reversed-phase Vydac 218TP54 C18-Säule durchgeführt wurde, bestimmt. Die Histone wurden bei -20°C eingefroren und lyophylisiert. Für die Cytotoxizität-Assays wurden die Histon-Präparationen in sterilem und Endotoxin-freiem destillierten Wasser bis zu einer Endkonzentration von 10 mg pro ml verdünnt. Für weitere Verdünnungen wurde RPMI, enthaltend 10% FCS, verwendet.

### Vergleichsbeispiel 1: Extraktion von H1-Histonen aus Rinderthymus.

Gefrorene Rinderthymuszellen wurden bei 4°C in 50 mM Tris/HCl, pH 7,5; 5 mM MgCl₂, 5 mM DTT und 250 µm PMSF mit einem Waring-Mischer homogenisiert. Nach der Zentrifugation bei 1.500 x g wurden die rohen Kempellets mit 0,75 M Perchlorsäure für eine Dauer von 2 Stunden unter kontinuierlichem Rühren extrahiert (Pehrson, J.R. and Cole, R.D. (1981) Biochemistry 20, 2298-2301). Die Mischung wurde wie in Beispiel 1 beschrieben gereinigt und direkt der HPLC unterzogen.

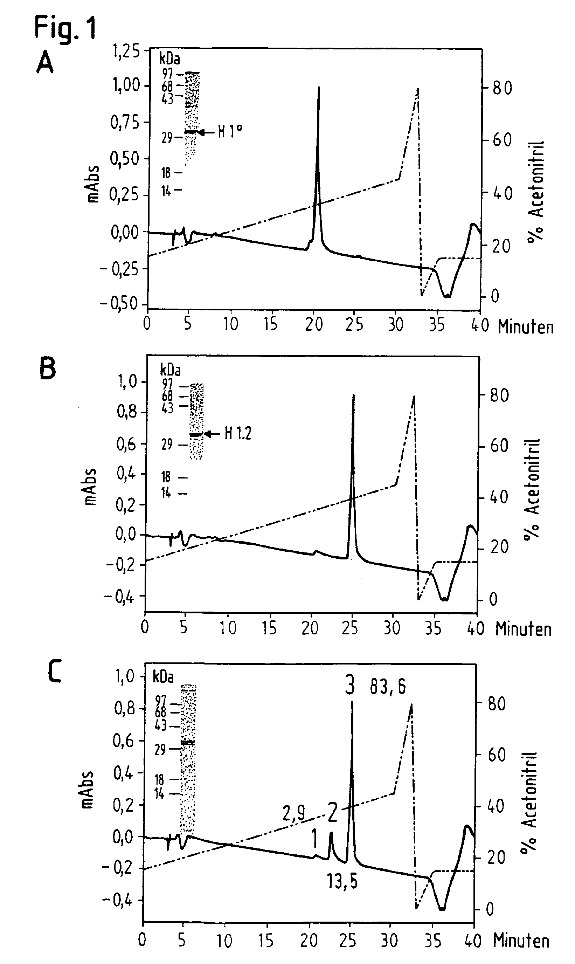

Die in der SDS-PAGE erhaltenen Fraktionen 3 bis 6, die nur zwei gemeinsam mit dem 30 kDa-Standard auftretende Hauptbanden enthielten, wurden gesammelt und als Rinderhiston H1 bezeichnet (siehe Abbildung 1C). Die analytische HPLC ergab zwei Hauptpeaks und einen geringeren Peak (siehe Abbildung 1C). Der erste Peak stellt Histon 1° mit einem berechneten Gehalt von 2,9% dieser Präparation dar. Der zweite Peak, der 13,5% der Proteine darstellt, konnte weder H1° noch H1.2 zugeordnet werden. Der Hauptpeak, der 83,6% der Proteine darstellt, konnte durch seine Retentionszeit H1.2 zugeordnet werden. Deswegen wird angenommen, dass H1.2 verantwortlich für die cytotoxischen Effekte von Rinderhiston 1 ist.

### Beispiel 2: Bestimmung der Cytotoxizität der H1°- und H1.2-Histone aus E. coli sowie der H1-Histone aus Rinderthymus auf die Leukämie-Zelllinie K562 und auf PBMCs.

Die PBMCs wurden aus Buffy Coats (Schicht der weißen Zellen, die sich zwischen der Schicht der roten Zellen und dem Plasma bildet, wenn nicht verklumptes Blut zentrifugiert wird oder stehengelassen wird) gesunder Spender durch Ficoll Hypaque (Sigma, Deisenhofen, Germany) isoliert, dreimal mit PBS gewaschen und in einer Dichte von 5 x 10⁵/ml in 10% FCS enthaltendem RPMI resuspendiert.

Die K562-Zellen wurden in RPMI mit 10% FCS gezüchtet. Die Zellen wurden bei logarithmischem Zellwachstum geerntet, einmal gewaschen und in einer Dichte von 5 x 10⁵/ml gelagert.

Für die Cytotoxizität-Assays wurden die PBMCs und die K562-Zellen in einer Konzentration von 5 x 10⁴ Zellen/Well in einer 96-Well-Flachbodenplatte ohne und mit unterschiedlichen Konzentrationen an Histonen inkubiert.

Die Cytotoxizität wurde durch Messung der Propidiumiodid-Inkorporation mit einem EPICS XL-Durchflußcytometer bestimmt (Coulter & Beckmann, Hamburg, Deutschland). Am Ende der Inkubation wurden die Zellen resuspendiert, und 200 µl der Zellsuspension wurden mit 200 µl Propidiumiodidlösung bis zu einer Endkonzentration von 2,5 µg pro ml Propidiumiodid (Sigma, Deisenhofen, Deutschland) vermischt. Der prozentuale Anteil von Zellen, die Propidiumiodid inkorporiert haben, von insgesamt 10.000 gezählten Zellen wurde unmittelbar nach der Zugabe der Propidiumiodidlösung bestimmt und als prozentuale Toxizität bezeichnet.

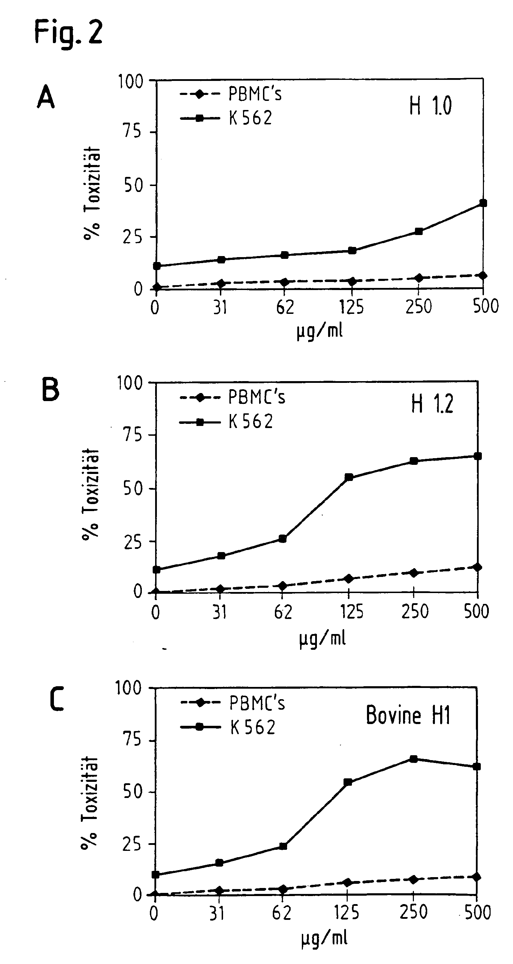

Nach einer Stunde Inkubation induzierten alle drei Histon-Präparationen, d.h. die rekombinant in *E*. *coli* hergestellten Histone H1° und H1.2 sowie das aus Rinderthymus isolierte Histon, eine signifikante und dosisabhängige Toxizität auf die Leukämie-Zelllinie K562 (Abbildung 2A, B, C durchgezogene Linien).

H1° wies bei Erhöhung der Histonkonzentration auf 125 µg/ml einen schwachen Anstieg der Cytoxizität und einen stärkeren Anstieg bei bis zu 250 und 500 µg/ml Histon auf. Bei dieser Konzentration starben 35% der Leukämiezellinien ab (Abbildung 2A).

H1.2 und das Rinderhiston 1 zeigten eine stark ansteigende Toxizität im Bereich von 62 µg/ml bis 125 µg/ml, in dem 63% bzw. 65% der Tumorzellen abstarben (Abbildung 2B, C). Ein weiterer Anstieg der Histonkonzentration auf 500 µg pro ml führten zu keiner (H1.2) bzw. zu einer nur geringen (Rinderhiston 1) Erhöhung der Cytotoxizität (siehe Abbildung 2B, C).

Die cytotoxischen Effekte auf PBMCs waren weniger ausgeprägt (siehe Abbildung 2A, B, C, gestrichelte Linien). Nach einer Stunde Inkubation mit 500 µg/ml Histon H1° waren noch 93,3% der Zellen lebensfähig (siehe Abbildung 2A). Unter den selben experimentellen Bedingungen überlebten 87,4% bzw. 91% der PBMCs die Inkubation mit rekombinantem H1.2 und Rinder H1-Präparation (siehe Abbildungen 2B, C).

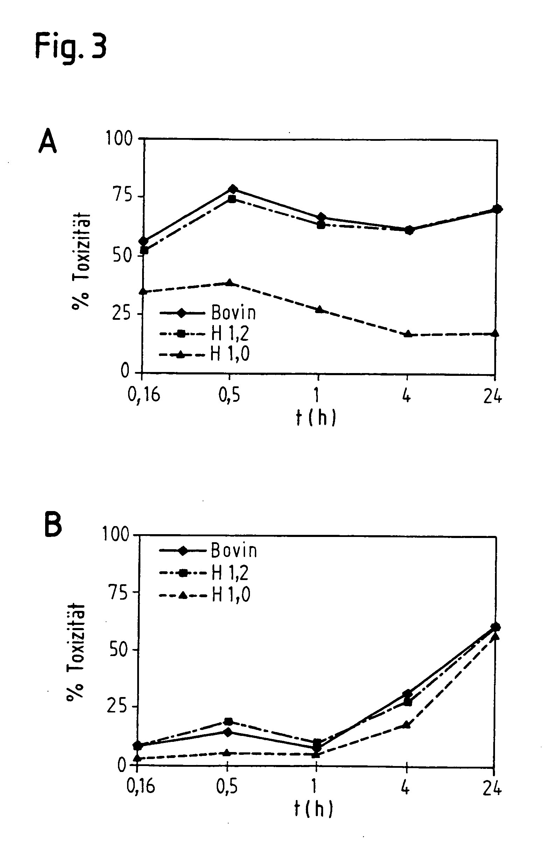

Die Zeitabhängigkeit der Histon-Cytotoxizität wurde jeweils bei 250 µg/ml Histon gemessen. Die Tumorzellcytotoxizität aller drei Histon-Präparationen begann unmittelbar nach Beginn der Inkubation und erreichte ihr Maximum während den ersten 30 Minuten der Inkubation (siehe Abbildung 3A). Im Fall von Histon H1.2 und dem Rinderhiston H1 waren innerhalb der ersten 10 Minuten der Inkubation 52,8% bzw. 56,3% der Leukämiezellen tot. Nach 30 Minuten stieg die Zahl der mit Propidiumiodid gefärbten Zellen bis zu 74,5% bzw. 78,6%. Im Fall von H1° wurde eine Toxizität von 35,2% innerhalb der ersten 10 Minuten beobachtet, die nach 30 Minuten lediglich um weitere 11% anstieg.

Die Toxizität von H1.2 und Rinderhiston für Tumorzelllinien blieb über einen Zeitraum von 24 Stunden annähernd stabil, während die Toxizität von H1° während desselben Zeitraums abnahm.

Im Gegensatz zu den Ergebnissen für Tumorzellen wiesen die humanen Histone H1° und H1.2 sowie das Rinderhiston 1 innerhalb der ersten Stunde der Inkubation keine signifikante Toxizität auf PBMCs auf (siehe Abbildung 3B). Nach 4 und 24 Stunden stieg die Toxizität jedoch auf Werte an, die vergleichbar zu der Toxizität auf K562-Zellen waren.

### Abbildungen

Abbildung 1: Reinheit der Histone. Aliquots von rekombinantem H1° (A), H1.2 (B) und Rinderhiston 1 (C) wurden mit analytischer Reversed-phase HPLC rechromatographiert. 2 µg von jedem Histon wurden durch SDS-PAGE auf einen 15%-Gel analysiert. Die mit Coomassie-Blue angefärbten Gele sind gezeigt. Die Hauptpeaks in (c) sind mit 1,2 und 3 entsprechend ihrer Retentionszeit bezeichnet.

Abbildung 2: Dosisabhängige Cytotoxizität der Histone. Die Cytotoxizität der rekombinanten Histone H1° (A), H1.2 (B) und von Rinderhiston H1 (C) auf PBMCs sowie auf die Leukämie-Zelllinie K562 (humane chronische myeloide Leukämie; Lozzio et al. (1973) J. Natl. Cancer Inst. 50:535-538; Zozzio et al. (1975) Blood 45:321-334) wurde nach 60 Minuten durch PI (Propidiumiodid)-Anfärbung gemessen.

Abbildung 3: Zeitabhängigkeit der Histon-Cytotoxizität. Die Leukämie-Zellinie K562 (a) und PBMCs (b) wurden mit 250 µg/ml humanem rekombinantem H1° bzw. H1.2 und Rinderhiston H1 inkubiert. Der Grad an Toxizität wurde durch PI-Anfärbung gemessen.

## Patentansprüche

1. Verfahren zur Herstellung von rekombinantem humanen Protein Histon 1, umfassend die folgenden Schritte:
a) Expression einer für humanes Protein Histon 1 kodierenden Nukleinsäuresequenz in prokaryoten Zellen, und
b) Extraktion und Reinigung des rekombinanten humanen Proteins Histon 1 aus den prokaryoten Zellen.

2. Verfahren nach Anspruch 1, wobei die prokaryote Zelle *E. coli* ist.

3. Verfahren nach Anspruch 1 oder 2, wobei das Histon 1 H1°oder H1.2 ist.

4. Verfahren gemäß einem der Ansprüche 1 bis 3, worin das Histon 1 H1.2 ist.

5. Verfahren zur Herstellung eines Therapeutikums auf der Basis von rekombinantem humanen Protein Histon H1 unter Durchführung des Verfahrens nach einem der Ansprüche 1 bis 4.

6. Verfahren nach Anspruch 5, wobei das Therapeutikum zur Krebstherapie eingesetzt werden kann.

7. Verfahren nach Anspruch 5 oder 6, wobei das Therapeutikum zur Therapie von Karzinomen, Melanomen, Sarkomen, Mesotheliomen und malignen Erkrankungen, insbesondere von solchen des lymphatischen Systems, die durch maligne B- und T-Zellen verursacht werden, wie B-lymphoblastisches Lymphom, myelogenisches Lymphom oder Burkitt-Lymphom eingesetzt werden kann.

8. Verfahren nach Anspruch 5, wobei das Therapeutikum ein Antibiotikum ist.

9. Verfahren nach Anspruch 5, wobei das Therapeutikum zur Immuntherapie, insbesondere zur Therapie von Autoimmunkrankheiten eingesetzt werden kann.

10. Verfahren nach Anspruch 5, wobei das Therapeutikum zur Therapie von endokrinen Störungen eingesetzt werden kann.

## Claims

1. Method of producing recombinant human protein histone 1, comprising the following steps:
a) expression of a nucleic acid sequence coding for human protein histone 1 in prokaryotic cells, and
b) extraction and purification of the recombinant human protein histone 1 from the prokaryotic cells.

2. Method according to claim 1, where the prokaryotic cell is *E. coli.*

3. Method according to claim 1 or 2, where the histone 1 is H1° or H1.2.

4. Method according to any of claims 1 to 3, where the histone 1 is H1.2.

5. Method of producing a therapeutic agent based on recombinant human protein histone 1 by carrying out the method of any of claims 1 to 4.

6. Method according to claim 5, where the therapeutic agent is used for cancer therapy.

7. Method according to claim 5, where the therapeutic agent is used for the therapy of carcinomas, melanomas, sarcomas, mesotheliomas and malignant diseases, in particular of malignant diseases of the lymphatic system, caused by malignant B- and T-cells, such as B-lymphoblastic lymphoma, myelogenic lymphoma or Burkitt-lymphoma.

8. Method according to claim 5, where the therapeutic agent is an antibiotic.

9. Method according to claim 5, where the therapeutic agent is used for immunotherapy, in particular for the therapy of autoimmune diseases.

10. Method according to claim 5, where the therapeutic agent is used for the therapy of endocrine disorders.

## Revendications

1. Procédé pour la production de la protéine humaine recombinante histone 1, comprenant les étapes suivantes :
a) l'expression d'une séquence d'acide nucléique codant la protéine humaine histone 1 dans les cellules procaryotes, et
b) l'extraction et la purification de la protéine humaine recombinante histone 1 à partir des cellules procaryotes.

2. Procédé selon la revendication 1, dans lequel la cellule procaryote est *E. coli.*

3. Procédé selon la revendication 1 ou 2, dans lequel l'histone 1 est H1 ou H1.2.

4. Procédé selon l'une des revendications 1 à 3, où l'histone 1 est H1.2.

5. Procédé pour la production d'un médicament sur la base de la protéine humaine recombinante histone 1 en mettant le procédé en oeuvre selon l'une des revendications 1 à 4.

6. Procédé selon la revendication 5, dans lequel le médicament peut être employé dans le cadre d'une thérapie anticancéreuse.

7. Procédé selon la revendication 5 ou 6, dans lequel le médicament peut être employé dans le cadre de la thérapie de carcinomes, de mélanomes, de sarcomes, de mésothéliomes et de maladies malignes, en particulier de ceux du système lymphatique qui sont provoqués par les cellules B et T malignes, comme le lymphome lymphoblastique B, le lymphome myélogène ou le lymphome de Burkitt.

8. Procédé selon la revendication 5, dans lequel le médicament est un antibiotique.

9. Procédé selon la revendication 5, dans lequel le médicament peut être employé dans le cadre de l'immunothérapie, en particulier dans le cadre de la thérapie des maladies auto-immunes.

10. Procédé selon la revendication 5, dans lequel le médicament peut être employé dans le cadre de la thérapie des désordres endocriniens.
